# EUROPEAN PATENT SPECIFICATION

(11) **EP 0 367 747 B1**
(45) Date of publication and mention of the grant of the patent: **13.03.1996**
(21) Application number: 89870169.3
(22) Date of filing: 31.10.1989
(51) Int. Cl.: C07D 207/12, A61K 31/40

(54) **Dideoxy-L-arabinitol derivatives as antiviral compounds**
Dideoxy-L-arabinitol Derivate als Antivirus-Verbindungen
dérivés du didéoxy-L-arabinitol en tant que composés antiviraux

(30) Priority: 03.11.1988 US 266718; 26.09.1989 US 410638; 26.09.1989 US 410640
(43) Date of publication of application: 09.05.1990
(73) Proprietor: G.D. Searle & Co., Skokie Illinois 60077 (US)
(72) Inventor: Koszyk, Francis Jan, Chicago Illinois 60641 (US); Partis, Richard Allen, Evanston Illinois 60201 (US); Mueller, Richard August, Glencoe Illinois 60022 (US)
(74) Representative: Ernst, Hubert

(56) References cited:
- EP-A- 0 322 395
- TETRAHEDRON LETTERS, vol. 26, no. 26, 1985, pages 3127-3130, Pergamon Press Ltd, GB; G.W.J. FLEET et al.: "Potent competitive inhibition of alpha-galactosidase and alpha-glucosidase activity by 1,4-dideoxy-1,4-iminopentitols:synthesis of 1,4-dideoxy-1,4-imino-D-lyxitol and of both enantiomers of 1,4-dideoxy-1,4-iminoarabinitol"
- TETRAHEDRON, vol. 42, no. 20, 1986, pages 5685-5692, Pergamon Journals Ltd, GB; G.W.J. FLEET et al.: "The synthesis from D-xylose of the potent and specific enhantiomeric glucosidase inhbitors, 1,4-dideoxy-1,4-imino-D-arabinitol and 1,4-dideoxy-1,4-imino-L-arabinitol"

## Description

This invention relates to novel antiviral compounds and intermediates for their synthesis and, more particularly, to N-alkyl, N-hydroxyalkyl, N-alkanoyl and N-aryl derivatives of 1,4-dideoxy-1,4-imino-L-arabinitol and their acylated derivatives. These compounds are inhibitors of visna virus, a pathogenic virus for sheep and goats. These antiviral compounds also have potential use for the treatment of acquired immune deficiency syndrome (AIDS) and AIDS-related complex (ARC).

Acquired immune deficiency syndrome, which only a few years ago was a medical curiosity, is now a serious disease. As a consequence, a great effort is being made to develop drugs and vaccines to combat AIDS. The AIDS virus, first identified in 1983, has been described by several names. It is the third known T-lymphocyte virus (HTLV-III) and has the capacity to replicate within cells of the immune system and thereby lead to a profound destruction of T4⁺ T-cells (or CD4⁺ cells). See, e.g., Gallo et al., Science 224, 500-503 (1984), and Popovic et al., Ibid., 497-500 (1984). This retrovirus has been known as lymphadenopathy-associated virus (LAV) or AIDS-related virus (ARV) and, most recently, as human immunodeficiency virus (HIV). Two distinct AIDS viruses, HIV-1 and HIV-2, have been described. HIV-1 is the virus originally identified in 1983 by Montagnier and co-workers at the Pasteur Institute in Paris [Ann. Virol. Inst. Pasteur 135 E, 119-134 (1984)], while HIV-2 was more recently isolated by Montagnier and his coworkers in 1986 [Nature 326, 662 -669 (1987)]. As used herein, HIV is meant to refer to these viruses in a generic sense.

Although the molecular biology of AIDS is beginning to be unraveled and defined, much more needs to be learned and understood about this disease. In the meantime, numerous approaches are being investigated in the search for potential anti-AIDS drugs and vaccines. Development of an AIDS vaccine is hampered by lack of understanding of mechanisms of protective immunity against HIV, the magnitude of genetic variation of the virus, and the lack of effective animal models for HIV infection. See, for example, Koff and Hoth, Science 241, 426-432 (1988).

The first drug to be approved by the U.S. Food and Drug Administration (FDA) for treatment of AIDS was zidovudine, better known under its former name azidothymidine (AZT). Chemically, this drug is 3′-azido-3′-deoxythymidine. This drug was originally selected as a potential weapon against AIDS because it was shown to inhibit replication of the virus in vitro. Such in vitro tests are useful and virtually the only practical method of initially screening and testing potential anti-AIDS drugs. A serious drawback of AZT, however, is its toxic side-effects. Thus, the search for better anti-AIDS drugs continues.

The HIV inhibitory activity of 1,4-dideoxy-1,4-imino-L-arabinitol and its N-methyl derivative is disclosed by Fleet et al., FEBS Lett. 237, 128-132 (1988).

### Brief Description of the Invention

In accordance with the present invention novel N-alkyl, N-hydroxyalkyl, N-alkanoyl and N-aryl derivatives of 1,4-dideoxy-1,4-imino-L-arabinitol and their acylated derivatives have been found to have useful antiviral activity.

1,4-dideoxy-1,4-imino-L-arabinitol is a five-membered heterocyclic compound having nitrogen in the ring and 3 hydroxyl groups. It is thus described by a systematic chemical name as a sugar derivative in which the five-membered ring is considered as a mimic of furanose, with nitrogen instead of oxygen in the ring. It can also be described structurally as a derivative of pyrrolidine. It can be prepared by joining the C-1 and C-4 of xylose together with nitrogen to form the pyrrolidine ring as described by Fleet and Smith, Tetrahedron 42, 5685-5692 (1986), or from xylitol in which only hydroxyl groups from C-1 and C-4 of xylose are left unprotected as disclosed by Fleet et al., Tetrahedron Lett. 26, 3127-3130 (1985).

The structure of the novel N-alkyl, N-hydroxyalkyl, N-alkanoyl and N-aryl derivatives of 1,4-dideoxy-1,4-imino-L-arabinitol and their acylated derivatives can be represented as follows: 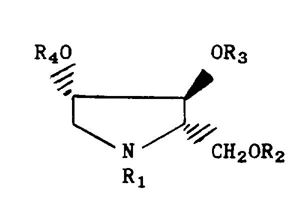 wherein R₁ is C₁-C₁₄ alkyl or C₁-C₁₄ hydroxyalkyl or C₃-C₁₂ alkanoyl or a substituted or unsubstituted aryl radical having six to ten carbon atoms; and
wherein R₂, R₃ and R₄ are the same or different and each is H or acyl having one to six carbon atoms; provided, however, that when R₂, R₃ and R₄ are each H, then R₁ is C₄-C₉ alkyl or C₂-C₅ hydroxyalkyl or C₃-C₁₂ alkanoyl.

Various of these novel compounds are useful intermediates for the preparation of acylated derivatives thereof which have antiviral activity. In the acylated derivatives, all the free hydroxyl groups are acylated with acyl groups having from one to six carbon atoms.

Typical compounds according to the invention are compounds of the general formula defined hereinbefore wherein R₁ is selected from the group consisting of alkyl and hydroxyalkyl as well as such compounds wherein, furthermore, R₂, R₃ and R₄ represent each the same acyl group.

Illustrative examples of the novel N-alkyl, N-hydroxyalkyl and N-alkanoyl derivatives of 1,4-dideoxy-1,4-imino-L-arabinitol are the following compounds:
1,4-(Butylimino)-1,4-dideoxy-L-arabinitol,
1,4-(Pentylimino)-1,4-dideoxy-L-arabinitol,
1,4-(Hexylimino)-1,4-dideoxy-L-arabinitol,
1,4-(Heptylimino)-1,4-dideoxy-L-arabinitol,
1,4-(2-Ethylbutylimino)-1,4-dideoxy-L-arabinitol,
1,4-(Octylimino)-1,4-dideoxy-L-arabinitol,
1,4-(Nonylimino)-1,4-dideoxy-L-arabinitol,
1,4-([2-Hydroxyethyl]imino)-1,4-dideoxy-L-arabinitol,
1,4-([3-Hydroxypropyl]imino)-1,4-dideoxy-L-arabinitol,
1,4-([4-Hydroxybutyl]imino)-1,4-dideoxy-L-arabinitol,
1,4-([5-Hydroxypentyl]imino)-1,4-dideoxy-L-arabinitol,
1,4-([2-Acetyloxyethyl]imino)-1,4-dideoxy-L-arabinitol,
1,4-([5-Acetyloxypentyl]imino)-1,4-dideoxy-L-arabinitol,
1,4-(Propionylimino)-1,4-dideoxy-L-arabinitol,
1,4-(2-Methylpropionylimino)-1,4-dideoxy-L-arabinitol.

Illustrative examples of antiviral compounds which can be made by acylation of the foregoing intermediates are the following compounds:
1,4-(Butylimino)-1,4-dideoxy-L-arabinitol, triacetate,
1,4-(Hexylimino)-1,4-dideoxy-L-arabinitol, triacetate,
1,4-(Nonylimino)-1,4-dideoxy-L-arabinitol, triacetate,
1,4-(Butylimino)-1,4-dideoxy-L-arabinitol, tributyrate,
1,4-(Butylimino)-1,4-dideoxy-L-arabinitol, tripropionate,
1,4-([5-Hydroxypentyl]imino)-1,4-dideoxy-L-arabinitol, triacetate,
1,4-([2-Acetyloxyethyl]imino)-1,4-dideoxy-L-arabinitol, triacetate,
1,4-([2-Acetyloxyethyl]imino)-1,4-dideoxy-L-arabinitol, tributyrate,
1,4-([5-Acetyloxypentyl]imino)-1,4-dideoxy-L-arabinitol, triacetate,
1,4-([5-Acetyloxypentyl]imino)-1,4-dideoxy-L-arabinitol, tributyrate,
1,4-(Propionylimino)-1,4-dideoxy-L-arabinitol, triacetate, and
1,4-(2-Methylpropionylimino)-1,4-dideoxy-L-arabinitol, triacetate.

Preferred method of preparation of the N-alkyl, N-hydroxyalkyl and N-alkanoyl substituted amine compounds is via catalytic reductive amination of the corresponding aldehyde or hydroxyaldehyde equivalent or by hydrogen transfer reductive alkylation. The hydroxyaldehyde equivalent may exist in a hemiacetal structure. Hydroxyaldehydes or their equivalents can be used where the hydroxyl group is protected, followed by removal of the protecting group. Hydride reduction is a preferred method of reductive alkylation where catalytic conditions would cause destruction of the starting material or products or overreaction. Cyanoborohydride reagents are most convenient hydride donors when acid stability is beneficial, e.g., when a rapid equilibrium between various chemical intermediates is desired. Examples of such equilibria can be hydroxyalkylaldehyde to hemiacetal, aminoacetal to amine and aldehyde and aminoacetal to an amine containing a double bond.

Illustratively, the N-alkyl derivatives can be prepared by hydrogenation of the starting amine together with an appropriate aldehyde in suitable solvent medium in the presence of palladium black catalyst as described, e.g., by Fleet et al, FEBS Lett. 237, 128-132 (1988). In an alternative method, the amine is reduced with sodium cyanoborohydride in the presence of a molecular sieve instead of reduction with the palladium black hydrogenation catalyst.

The aryl radicals are illustrated, e.g., by phenyl, phenylacetyl, alkylphenyl, benzyl, benzoyl, benzyloxycarbonyl and naphthyl.

The aryl radicals can have one or more, preferably 1 to 3, identical or different substituents. Examples of substituents are alkyl or alkoxy having from one to ten carbon atoms; halogen such as Cl, Br or F; and hydroxyl.

Preferred alkyl moieties in the aryl radicals and in the acyl groups have from one to six carbon atoms such as methyl, ethyl, propyl, butyl and isobutyl.

Illustrative examples of these novel antiviral compounds are the following:
1,4-([4-Chlorophenyl]methylimino)-1,4-dideoxy-L-arabinitol,
1,4-([4-Chlorophenyl]-methylimino)-1,4-dideoxy-L-arabinitol, triacetate,
1,4-([4-Ethylphenyl]methylimino)-1,4-dideoxy-L-arabinitol,
1,4-([4-Ethylphenyl]methylimino)-1,4-dideoxy-L-arabinitol, triacetate,
1,4-([3-Propylphenyl]imino)-1,4-dideoxy-L-arabinitol,
1,4-([3-Propylphenyl]imino)-1,4-dideoxy-L-arabinitol, triacetate,
1,4-(Benzyloxycarbonylimino)-1,4-dideoxy-L-arabinitol,
1,4-(Benzyloxycarbonylimino)-1,4-dideoxy-L-arabinitol, triacetate,
1,4-(Benzylcarbonylimino)-1,4-dideoxy-L-arabinitol, tributyrate,
1,4-(Benzyloxycarbonylimino)-1,4-dideoxy-L-arabinitol, triisobutyrate,
1,4-(Benzyloxycarbonylimino)-1,4-dideoxy-L-arabinitol, tripropionate,
1,4-([Benzyloxypentyl]imino)-1,4-dideoxy-L-arabinitol,
1,4-([Benzyloxypentyl]imino)-1,4-dideoxy-L-arabinitol, triacetate,
1,4-(Benzoylimino)-1,4-dideoxy-L-arabinitol,
1,4-(Benzoylimino)-1,4-dideoxy-L-arabinitol, triacetate,
1,4-(Phenylacetylimino)-1,4-dideoxy-L-arabinitol, and
1,4-(Phenylacetylimino)-1,4-dideoxy-L-arabinitol, triacetate.

The novel antiviral compounds with N-aryl substituents can be prepared from the amine, 1,4-dideoxy-1,4-imino-L-arabinitol, by conventional N-alkylation with appropriate aryl groups. The free hydroxyl groups on the amine can be acylated either before or after this N-alkylation.

In preferred embodiments, reductive alkylation can be carried out by reaction of the starting amine with an appropriate arylaldehyde in the presence of sodium cyanoborohydride reducing agent and methanol solvent. Illustrative arylaldehydes are, e.g., benzaldehyde, chlorobenzaldehyde, ethylbenzaldehyde and hydrocinnamaldehyde.

Alternatively, reduction by palladium catalyzed hydrogenation followed by treatment with benzyl chloroformate can be carried out to give N-benzyloxycarbonyl derivatives of the amine. In this procedure, a novel intermediate, methyl 2,5-dideoxy-2,5-imino-α-L-lyxofuranoside, tosylate salt, is a useful starting point for the alkylation with benzyl chloroformate. This intermediate can be prepared by the palladium catalyzed hydrogenation of methyl 2-azido-2-deoxy-5-O-p-toluenesulfonyl-α-L-lyxofuranoside. The latter material is analogous to the corresponding D-isomer described by Fleet and Smith, Tetrahedron 42, 5685-5692 (1986).

Acylation of the free hydroxyl groups is conveniently carried out by reaction of the amine with an appropriate acid anhydride such as, e.g., the acetic-, propionic-, butyric- or isobutyric anhydrides.

In other preferred embodiments, the pre-acylated amine can be reacted with alkylating agents to form the N-aryl derivatives. Illustrative of such alkylating agents are, e.g., benzoyl chloride or phenylacetic anhydride together with triethylamine.

Although specific methods of production are described herein, it will be appreciated that the novel antiviral compounds claimed herein are not limited to any particular method of production.

The foregoing antiviral compounds can be demonstrated to have inhibitory activity against visna virus in a conventional plaque reduction assay. Visna virus, a lentivirus genetically very similar to the AIDS virus, is pathogenic for sheep and goats. See Sonigo et al., Cell 42, 369-382 (1985); Haase, Nature 322, 130-136 (1986). Inhibition of visna virus replication in vitro also is a useful model for human immunodeficiency virus (HIV), and its inhibition by test compounds has been described by Frank et al., Antimicrobial Agents and Chemotherapy 31 (9), 1369-1374 (1987).

The N-butyl derivative of 1,5-dideoxy-1,5-imino-D-glucitol, also referred to as N-butyl-deoxynojirimycin (N-Bu-DNJ), was used as a control standard for comparison with various novel compounds disclosed herein. The HIV inhibitory activity of N-Bu-DNJ is described in U.S. Patent 4,849,430.

Inhibitory activity can also be demonstrated by the acylated derivatives against alpha- and beta-glucosidase enzymes. In some cases, the non-acylated derivatives also have effective inhibitory activity against visna virus, cytomegalovirus (CMV) and/or the alpha- and beta- glucosidases.

### Detailed Description of the Invention

The following detailed examples will further illustrate the invention.

### Example 1

### A. 1,4-Dideoxy-1,4-imino-L-arabinitol hydrochloride

### B. 1,4-Dideoxy-1,4-imino-L-arabinitol

The title compounds were prepared by the method described by Fleet and Smith, Tetrahedron 42, 5685-5692 (1986), to prepare the D-isomers, except that L-xylose was used as the starting material instead of D-xylose.

### Example 2

### 1,4-Dideoxy-1,4-([2-hydroxyethyl]imino)-L-arabinitol

To a solution of the title product of Example 1A (1.44 g, 8.50 mmoles) in 25 ml of methanol was added a solution of sodium bicarbonate (714 mg, 8.50 mmoles) in 10 ml of water. After stirring for a few minutes, the solvent was removed on a rotary evaporator. The residue was then dissolved in anhydrous ethanol, and the solvent was removed on a rotary evaporator. The residue was dissolved in a mixture of 29 ml of methanol and 1.5 ml of acetic acid. To the resulting solution was added glycolaldehyde dimer (1.02 g, 8.50 mmoles), 5 g of 4Å molecular sieves, and then, in portions, sodium cyanoborohydride (553 mg, 8.81 mmoles). After stirring overnight at room temperature, the mixture was filtered, and the solvent was removed on a rotary evaporator. Chromatography of the residue on silica gel using 50-50 ethyl acetate-methanol as eluant gave the title compound (1.82 g) as an oil. The compound was identified by proton and carbon NMR spectrometry.

### Example 3

### 1,4-([2-Acetyloxyethyl]imino)-1,4-dideoxy-L-arabinitol, triacetate

To a solution of the title product of Example 2 (343 mg, 1.9 mmoles) in 10 ml of pyridine was added 4 ml of acetic anhydride. The residue was stirred for one hour at room temperature, and then at reflux for 5 minutes. After cooling, the mixture was poured into 30 ml of ice water and extracted with three portions of ethyl acetate. The combined organic extracts were washed with 25 ml of dilute hydrochloric acid, dried over sodium sulfate, filtered, and the solvent removed on a rotary evaporator. Chromatography of the residue on silica gel using a gradient of 50 to 75% ethyl acetate-hexane as eluant gave the title compound (418 mg) as an oil.
Analysis for C₁₅H₂₃NO₈ (MW 345.35):
Calcd.: C, 52.17; H, 6.71; N, 4.06.
Found: C, 51.77; H, 6.66; N, 4.00.

### Example 4

### 1,4-(Butylimino)-1,4-dideoxy-L-arabinitol

The title compound (822 mg) was prepared as an oil by the method of Example 2 by using N-butyraldehyde (1.27 g) instead of glycolaldehyde dimer, and by using 1.50 g of the product of Example 1A as the starting material. The title compound was identified by proton and carbon NMR spectrometry.

### Example 5

### 1,4-(Butylimino)-1,4-dideoxy-L-arabinitol, triacetate

The title compound (418 mg) was prepared as an oil by the method of Example 3, using the product of Example 4 instead of the product of Example 2 as the starting material, and using 35% ethyl acetate-hexane as the chromatography eluant.
Analysis for C₁₅H₂₅NO₆ (MW 315.37):
Calcd.: C, 57.13; H, 7.99; N, 4.44.
Found: C, 56.84; H, 7.85; N, 4.42.

### Example 6

### 1,4-(Hexylimino)-1,4-dideoxy-L-arabinitol

To a solution of the title product of Example 1B (250 mg, 1.88 mmoles) in a mixture of 6.3 ml of methanol and 0.3 ml of acetic acid was added 1.1 g of 4Å molecular sieves, n-hexanal (377 mg, 3.76 mmoles) and then, in portions, sodium cyanoborohydride (123 mg, 1.96 mmoles). After stirring overnight at room temperature, the mixture was filtered, and the solvent was removed on a rotary evaporator. Chromatography of the residue on silica gel using 15% methanol - 2.5% ammonium hydroxide - 82.5% ethyl acetate as eluant gave a partially purified product. The material was dissolved in 5 ml of 50-50 trifluoroacetic acid-water. After 15 minutes the solvent was removed on a rotary evaporator. The residue was dissolved in water, passed through a basic ion exchange resin, and eluted with water. Appropriate fractions were then passed through an acidic ion exchange resin and eluted with aqueous ammonium hydroxide. Appropriate fractions were lyophilized to give the title compound (123 mg) as an oil.
Analysis for C₁₁H₂₃NO₃ (MW 217.31):
Calcd. : C, 60.79; H, 10.67; N, 6.45.
Found : C, 60.36; H, 10.50; N, 6.35.

### Example 7

### 1,4-(Hexylimino)-1,4-dideoxy-L-arabinitol, triacetate

The title compound (56 mg) was prepared as an oil by the method of Example 3, using the product of Example 6 (64 mg) as the starting material, and using 25% ethyl acetate-hexane as the chromatography eluant.
Analysis for C₁₇H₂₉NO₆ (MW 343.42):
Calcd.: C, 59.46; H, 8.51; N, 4.08.
Found: C, 59.13; H, 8.50; N, 4.04.

### Example 8

### 1,4-([4-Chlorophenyl]methylimino)-1,4-dideoxy-L-arabinitol

To a solution of the title product of Example 1B (447 mg, 3.36 mmole) in a mixture of 11 ml of methanol and 0.5 ml of acetic acid was added 2.0 g of 4Å molecular sieves, 948 mg (6.72 mmoles) of 4-chlorobenzaldehyde, and then, in portions, 220 mg (3.49 mmoles) of sodium cyanoborohydride. After stirring overnight at room temperature the mixture was filtered, and the solvent removed on a rotary evaporator. Chromatography of the residue over silica gel using 10% methanol - 2.5% ammonium hydroxide - 87.5% ethyl acetate as eluant followed by crystallization from ethyl acetate-hexane gave the title compound (189 mg) as a white solid, m.p. 94°C.
Analysis for C₁₂H₁₆CINO₃ (MW 257.72):
Calcd.: C, 55.92; H, 6.25; N, 5.44.
Found: C, 55.54; H, 6.21; N, 5.44.

### Example 9

### 1,4-([4-Ethylphenyl]methylimino)-1,4-dideoxy-L-arabinitol

The title compound (190 mg) was prepared as a waxy solid by the method of Example 6 by using 4-ethylbenzaldehyde (785 mg) instead of hexanal, using 390 mg of the product of Example 1B, and by using 25% methanol - 2.5% ammonium hydroxide - 72.5% ethyl acetate as the chromatography eluant.
Analysis for C₁₄H₂₁NO₃ . 1/8 H₂O (MW 253.55):
Calcd.: C, 66.32; H, 8.45; N, 5.52.
Found: C, 66.29; H, 8.29; N, 5.46.

### Example 10

### 1,4-([4-Ethylphenyl]methylimino)-1,4-dideoxy-L-arabinitol, triacetate

The title compound (132 mg) was prepared as an oil by the method of Example 3, using the product of Example 9 (87 mg) instead of the product of Example 2 as the starting material, and using 50-50 ethyl acetate-hexane as the chromatography eluant.
Analysis for C₂₀H₂₇NO₆ (MW 377.44):
Calcd.: C, 63.65; H, 7.21; N, 3.71.
Found: C, 63.59; H, 7.37; N, 3.61.

### Example 11

### 1,4-(Nonylimino)-1,4-dideoxy-L-arabinitol

The title compound was prepared by the method of Example 2 using nonanal (557 mg) instead of glycolaldehyde dimer, 333 mg of the product of Example 1A, and 15% methanol - 2.5% ammonium hydroxide -82.5% ethyl acetate as the chromatography eluant. The product, characterized by spectral methods, was used below without further purification.

### Example 12

### 1,4-(Nonylimino)-1,4-dideoxy-L-arabinitol, triacetate

The title compound was prepared by the method of Example 3, using the product of Example 11 instead of the product of Example 2 as the starting material, and using a gradient of 20 to 30% ethyl acetate-hexane as the chromatography eluant.
Analysis for C₂₀H₃₅NO₆ (MW 385.51):
Calcd.: C, 62.31; H, 9.15,; N, 3.63.
Found: C, 62.42; H, 8.80; N, 3.51.

### Example 13* (*synthesis of intermediate)

### 2 -Hydroxytetrahydropyran** (**intermediate product)

Dihydropyran (23 g, 274 mmoles) was added to 100 ml of 0.2 molar aqueous hydrochloric acid and the resulting mixture was stirred at room temperature. The mixture was then neutralized with dilute aqueous sodium hydroxide solution. Distillation at reduced pressure through a Vigreux column gave the title compound as a water-white liquid. ¹H and ¹³C NMR spectra confirmed the structure of the compound.

### Example 14

### 1,4-([5-Hydroxypentyl]imino)-1,4-dideoxy-L-arabinitol

The title compound (360 mg) was prepared by the method of Example 6 by using the product of Example 13 (897 mg) instead of n-hexanal, and by using 390 mg of the product of Example 1B.
Analysis for C₁₀H₂₁NO₄ . ¼ H₂O (M.W. 223.80):
Calcd.: C, 53.66; H, 9.68; N, 6.28.
Found: C, 53.60; H, 9.82; N, 6.23.

### Example 15

### 1,4-([5-Acetyloxypentyl]imino)-1,4-dideoxy-L-arabinitol, triacetate

The title compound was prepared as an oil by the method of Example 3 by using the product of Example 14 instead of the product of Example 2.
Analysis for C₁₈H₂₉NO₈ (MW 387.43):
Calcd.: C, 55.81; H, 7.55; N, 3.62.
Found: C, 55.75; H, 7.47; N, 3.51.

### Example 16

### 1,4-([3-Propylphenyl]imino)-1,4-dideoxy-L-arabinitol

A solution of the title product of Example 1B (250 mg, 1.88 mmoles) and hydrocinnamaldehyde (504 mg, 3.76 mmoles) in methanol was hydrogenated in the presence of 5% palladium and carbon at room temperature under a pressure of 5 pounds per square inch of hydrogen for 70 hours. The catalyst was filtered off and the solvent removed on a rotary evaporator. Chromatography of the residue on silica gel using 20% methanol - 2.5% ammonium hydroxide - 77.5% ethyl acetate as eluant followed by crystallization from toluene gave the title compound as a light tan crystalline solid (153 mg), m.p. 63°C.
Analysis for C₁₄H₂₁NO₃. 1/4 H₂O (MW 255.83):
Calcd.: C, 65.75; H, 8.47; N, 5.48.
Found: C, 65.64; H, 8.43; N, 5.52.

### Example 17

### 1,4-(Benzyloxycarbonylimino)-1,4-dideoxy-L-arabinitol

A. Methyl 2-azido-2-deoxy-5-O-p-toluene-sulfonyl-α-L-lyxofuranoside was prepared by the method described by Fleet and Smith, Tetrahedron 42, 5685-5692 (1986), for the preparation of the corresponding D-isomer except that L-xylose was used herein as the starting material instead of D-xylose.

B. Methyl 2,5-dideoxy-2,5-imino-α-L-lyxofuranoside, tosylate salt, was prepared from the azido tosylate product of Part A, above, by hydrogenation of a solution of 83 grams of said azido tosylate in ethanol in the presence of 5% palladium on carbon under a pressure of 5 pounds per square inch of hydrogen for 4-1/2 hours. The catalyst was filtered off and the solvent removed on a roatry evaporator. Crystallization of the residue from methylene chloride-methanol-ethyl acetate gave methyl 2,5-dideoxy-2,5-imino-α-L-lyxofuranoside, tosylate salt, as a pure white crystalline solid, m.p. 115-116°. The structure of this novel compound was confirmed by spectroscopic methods.

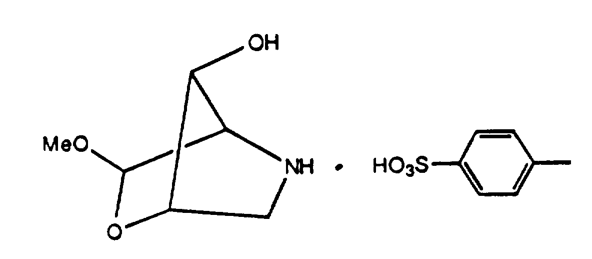

C. The title compound, 1,4-(Benzyloxycarbonylimino)-1,4-dideoxy-L-arabinitol, was prepared from the tosylate salt of Part B, above, as follows. To a mixture of 25.0 grams of said tosylate salt, 250 ml of ethyl acetate, and 125 ml of saturated aqueous sodium bicarbonate at 0° was added dropwise benzyl chloroformate (17.5 g) with rapid stirring. After 1.0 hour, the layers were separated, and the aqueous layer was extracted with ethyl acetate. The combined organic layers were dried over sodium sulfate, the drying agent was removed by filtration, and the filtrate was removed on a rotary evaporator. The pale yellow residue was then dissolved in a 4:1 mixture of trifluoroacetic acid and water (220 ml). After 0.5 hour at room temperature, the solvents were removed on a rotary evaporator and the residue was dried by azeotropic distillation with benzene. The residue was then dissolved in ethanol (225 ml), and a solution of sodium borohydride (2.24 g) in water (23 ml) was added dropwise. After stirring for 15 minutes at room temperature, ammonium chloride (2.25 g) was added, and the solvent removed on a rotary evaporator. The residue was partitioned between ethyl acetate and water, and the aqueous layer was extracted with four portions of ethyl acetate. The combined organic extracts were dried over sodium sulfate, the drying agent was removed by filtration and the solvent was removed on a rotary evaporator. Chromatography of the residue on silica gel using a gradient of 0-10% methanol-ethyl acetate gave the title compound, 1,4-(Benzyloxycarbonylimino)-1,4-dideoxy-L-arabinitol, (15.2 g) as a pale tan solid, m.p. 125-126.5°. The structure was confirmed by spectroscopic methods.

### Example 18 * (*comparative example)

### 1,4-(Methylimino)-l,4-dideoxy-L-arabinitol** (** comparative and intermediate product)

A mixture of the title product of Example 17, Part C (500 mg), 100 mg of 10% palladium on carbon, 17 ml of ethanol, and 2 ml of cyclohexene was stirred at reflux under a nitrogen atmosphere for 2.5 hours. After cooling, 430 µl of a 37% aqueous solution of formaldehyde was added and the resulting solution stirred overnight at room temperature. A further portion (50 mg) of 10% palladium on carbon and 1 ml of cyclohexene were added and stirring was continued at reflux for 6 hours. The solids were removed by filtration and the solvent removed on a rotary evaporator. Chromatography on silica gel using 40% methanol - 2.5% ammonium hydroxide - 57.5% ethyl acetate as eluant gave a partially purified product. The residue was passed through an acidic ion exchange resin and eluted with dilute aqueous ammonium hydroxide. The appropriate fractions were lyophilized to give the title compound as a very pale yellow oil (160 mg).
Analysis for C₆H₁₃NO₃. 3/8 H₂O (MW 153.94):
Calcd.: C, 46.83; H, 9.01; N, 9.10.
Found: C, 46.86; H, 9.08; N, 9.61.

### Example 19

### 1,4-(Methylimino)-1,4-dideoxy-L-arabinitol, triacetate

The title compound was prepared by the method of Example 3 using the product of Example 18 instead of the title product of Example 2. The compound was identified by ¹H NMR spectroscopy.

### Example 20

### 1,4-(Benzyloxycarbonylimino)-1,4-dideoxy-L-arabinitol, triacetate

To a solution of the title product of Example 17, Part C (1.00g) in 25 ml of pyridine was added 5 ml of acetic anhydride. After standing overnight at room temperature, the mixture was partitioned between ethyl acetate and water. The organic layer was washed successively with two portions of aqueous copper sulfate solution, water, and brine. After drying over anhydrous sodium sulfate, the solution was filtered, and the solvent was removed on a rotary evaporator. Chromatography of the residue on silica gel using 35% ethyl acetate-hexane as eluant gave the title compound as an oil.
Analysis for C₁₉H₂₃NO₈ (MW 393.40):
Calcd.: C, 58.01; H, 5.89; N, 3.56.
Found: C, 57.76; H, 5.78; N, 3.51.

### Example 21

### 1,4-Imino-1,4-dideoxy-L-arabinitol, triacetate

To a solution of the title product of Example 20 (3.00g) in a mixture of 72 ml of ethanol and 8 ml of cyclohexene was added 300 mg of 10% palladium on carbon. The resulting mixture was stirred at reflux for 5 hours under nitrogen and then cooled. The catalyst was removed by filtration and the solvent was removed on a rotary evaporator. Chromatography of the residue on silica gel using a gradient of 0% to 10% methanol - ethyl acetate gave the title compound as an oil. The structure was verified by ¹H NMR spectrometry.

### Example 22

### 1,4-(Benzyloxycarbonylimino)-1,4-dideoxy-L-arabinitol, tributyrate

The title compound was prepared by the method of Example 20 by using butyric anhydride instead of acetic anhydride, and by conducting the reaction at reflux for 0.5 hour instead of at room temperature.
Analysis for C₂₅H₃₅NO₈ (MW 477.56):
Calcd.: C, 62.88; H, 7.39; N, 2.93.
Found: C, 62.70; H, 7.44; N, 2.88.

### Example 23

### 1,4-Imino-1,4-dideoxy-L-arabinitol, tributyrate

The title compound was prepared by the method of Example 21 by using the product of Example 22 instead of the product of Example 20.
Analysis for C₁₇H₂₉NO₆ (MW 343.42):
Calcd.: C, 59.46; H, 8.51; N, 4.08.
Found: C, 59.06; H, 8.30; N, 4.02.

### Example 24

### 1,4-(Benzyloxycarbonylimino)-1,4-dideoxy-L-arabinitol, triisobutyrate

The title compound was prepared by the method of Example 20 by using isobutyric anhydride instead of acetic anhydride, by the addition of 4-dimethylaminopyridine in catalytic amount, and by using a gradient of 25% to 50% ethyl acetate-hexane as the chromatography eluant.
Analysis for C₂₅H₃₅NO₈ (MW 477.56):
Calcd.: C, 62.88; H, 7.39; N, 2.93.
Found: C, 62.53; H, 7.35; N, 2.91.

### Example 25

### 1,4-Imino-1,4-dideoxy-L-arabinitol, triisobutyrate

The title compound was prepared by the method of Example 21 by using the product of Example 24 instead of the product of Example 20.
Analysis for C₁₇H₂₉NO₆. 1/4 H₂O (MW 347.92):
Calcd.: C, 58.70; H, 8.55; N, 4.03.
Found: C, 58.63; H, 8.53; N, 4.38.

### Example 26

### 1,4-(Butylimino)-1,4-dideoxy-L-arabinitol, tributyrate

The title compound was prepared by the method of Example 20 by using the product of Example 4 instead of the product of Example 17, by using butyric anhydride instead of acetic anhydride, and by using 10% ethyl acetate-hexane as the chromatography eluant.
Analysis for C₂₁H₃₇NO₆ (MW 399.53):
Calcd.: C, 63.13; H, 9.34; N, 3.51.
Found: C, 63.34; H, 9.36; N, 3.47.

### Example 27

### 1,4-(Benzoylimino)-1,4-dideoxy-L-arabinitol, triacetate

To a solution of the title product of Example 21 (188 mg, 0.726 mmole) in 5 ml of dichloromethane was added triethylamine (146 mg, 1.45 mmoles) and then benzoyl chloride (123 mg, 0.871 mmole). After stirring overnight at room temperature, 25 ml of dichloromethane was added. The resulting solution was washed with dilute hydrochloric acid and with water. After drying over anhydrous sodium sulfate, the solution was filtered and the solvent removed on a rotary evaporator. Radial chromatography of the residue on silica gel using 75% ethyl acetate-hexane as eluant gave the title compound (222 mg) as an oil.
Analysis for C₁₈H₂₁NO₇ (MW 363.37):
Calcd.: C, 59.49; H, 5.83; N, 3.86.
Found: C, 59.26; H, 5.91; N, 3.72.

### Example 28

### 1,4-(Phenylacetylimino)-1,4-dideoxy-L-arabinitol, triacetate

To a solution of the title product of Example 21 (290 mg, 1.12 mmoles) in 7.5 ml of dichloromethane was added phenylacetic anhydride (341 mg, 1.34 mmoles), triethylamine (271 mg, 2.68 mmoles), and 2 mg of 4-dimethylaminopyridine. After stirring overnight at room temperature, the mixture was diluted with dichloromethane. The resulting solution was washed successively with water, aqueous sodium bicarbonate solution, dilute hydrochloric acid, and water. After drying over anhydrous sodium sulfate, the solution was filtered and the solvent removed on a rotary evaporator. Radial chromatography of the residue on silica gel using 75% ethyl acetate-hexane as eluant gave the title compound (249 mg) as a colorless oil.
Analysis for C₁₉H₂₃NO₇ (MW 377.40):
Calcd.: C, 60.47; H, 6.14; N, 3.71.
Found: C, 60.33; H, 6.21; N, 3.69.

### Example 29

### 1,4-(Benzyloxycarbonylimino)-1,4-dideoxy-L-arabinitol, tripropionate

The title compound was prepared by the method of Example 24 by using propionic anhydride instead of isobutyric anhydride.
Analysis for C₂₂H₂₉NO₈ (MW 435.48):
Calcd.: C, 60.67; H, 6.71; N, 3.22.
Found: C, 60.53; H, 6.70; N, 3.20.

### Example 30

### 1,4-Imino-1,4-dideoxy-L-arabinitol, tripropionate

The title compound was prepared by the method of Example 25 by using the product Example 29 instead of the product of Example 20.
Analysis for C₁₄H₂₃NO₆. 1/8 H₂O (MW 301.34):
Calcd.: C, 55.40; H, 7.72; N, 4.61.
Found: C, 55.39; H, 7.90; N, 4.67.

### Example 31

### 1,4-(Propionylimino)-1,4-dideoxy-L-arabinitol, triacetate

The title compound was prepared as a colorless oil by the method of Example 28 by using propionic anhydride instead of phenylacetic anhydride.
Analysis for C₁₄H₂₁NO₇ (MW 315.33):
Calcd.: C, 53.35; H, 6.71; N, 4.44.
Found: C, 52.97; H, 6.79; N, 4.33.

### Example 32

### 1,4-(2-Methylpropionylimino)-1,4-dideoxy-L-arabinitol, triacetate

The title compound was prepared as a colorless oil by the method of Example 28 by using isobutyric anhydride instead of phenylacetic acid.
Analysis for C₁₅H₂₃NO₇ . ¼ H₂O (MW 333.85):
Calcd.: C, 53.97; H, 7.09; N, 4.20.
Found: C, 54.06; H, 7.05; N, 4.22.

### Example 33

### 5-Benzyloxy-1-hexene

To a stirred mixture of sodium hydride (2.6g, 110 mmoles) in tetrahydrofuran (185 ml) under a nitrogen atmosphere was added a solution of 5-hexene-1-ol (10g, 100 mmoles) in tetrahydrofuran (15 ml). After stirring at room temperature for 0.5 hour, the mixture was briefly heated to reflux and then cooled. Benzyl bromide (21.4 g, 125 mmoles) was added, and the mixture was stirred at reflux for one hour. Stirring was continued overnight at room temperature, after which the mixture was concentrated on a rotary evaporator. The mixture was poured into water, and the aqueous layer was extraced with three portions of ether. The combined organic estracts were dried over sodium sulfate, filtered, and the solvent evaporated. Chromatography of the residue on silica gel using a gradient of 0 to 10% ethyl acetate-hexane as eluant gave the title product (11.2 g) as an oil. The structure was confirmed by ¹H NMR spectrometry.

### Example 34

### 5-Benzyloxy-1-pentanal

A solution of 5-benzyloxy-1-hexene prepared according to Example 33 (11.2 g, 58.9 mmoles) in dichloromethane (200 ml) at -70° was ozonized until a blue color persisted. The excess ozone was purged with a stream of oxygen gas, and then dimethyl sulfide (11.0 g, 177 mmoles) was added. After stirring overnight at room temperature, the volatiles were removed on a rotary evaporator. The residue was taken up in ether, washed with water and then brine, dried over sodium sulfate, filtered, and the solvent evaporated. Chromatography of the residue on silica gel using a gradient of 10 to 50% ethyl acetate-hexane gave the title compound (3.34 g) as an oil. The structure was confirmed by ¹H NMR spectrometry.

### Example 35

### 1,4-([5-Benzyloxypentyl]imino)-1,4-dideoxy-L-arabinitol

The title compound (689 mg) was prepared as an oil by the method of Example 6 except that 5-benzyloxy-1-pentanal prepared according to Example 34 (1.44 g) was used instead of hexanal, and the treatment with trifluoroacetic acid-water followed by ion exchange chromatography was omitted. The structure was confirmed by ¹H NMR spectrometry.

### Example 36

### 1,4-([5-Benzyloxypentyl]imino)-1,4-dideoxy-L-arabinitol, triacetate

The title compound (410 mg) was prepared as an oil by the method of Example 7 except that the product of Example 35 (564 mg) was used as the starting material. The structure was confirmed by ¹H NMR spectrometry.

### Example 37

### 1,4-([5-Hydroxypentyl]imino)-1,4-dideoxy-L-arabinitol, triacetate

A solution of the title product of Example 36 (410 mg) in absolute ethanol was hydrogenated in the presence of palladium black at 60° under a pressure of 60 pounds per square inch of hydrogen for hours. The catalyst was filtered off and the solvent removed on a rotary evaporator. Chromatography of the residue on silica gel using a gradient of 75 to 100% ethyl acetate-hexane as eluant gave the title compound (230 mg) as an oil.
Analysis for C₁₆H₂₇NO₇ (MW 345.40):
Calcd.: C, 55.65; H, 7.88; N, 4.06.
Found: C, 55.38; H, 7.91; N, 4.00.

### Example 38

Various compounds as prepared above were tested for inhibition of visna virus in vitro in a plaque reduction assay as follows:

### METHOD

### Cell and virus propagation

Sheep choroid plexus(SCP) cells were obtained from American Type Culture Collection (ATCC) catalogue number CRL 1700 and were routinely passaged in vitro in Dulbecco's Modified Eagles (DME) medium supplemented with 20% fetal bovine serum (FBS). SCP cells were passaged once per week at a 1:2 or 1:3 split ratio. Visna was titrated by plaque assay in six-well plates. Virus pools were stored at -70°C.

### Plaque reduction assay

SCP cells were cultured in 6-well plates to confluence. Wells were washed two times with serum free Minimal Essential Medium (MEM) to remove FBS. 0.2ml of virus was added per well in MEM supplemented with 4mM glutamine and gentamycin. After 1 hour adsorption, the virus was aspirated from each well. The appropriate concentration of each compound in 5 ml of Medium 199 (M-199) supplemented with 2% lamb serum, 4mM glutamine, 0.5% agarose and gentamycin was added to each well. Cultures were incubated at 37°C in a humidified 5% CO₂ incubator for 3-4 weeks. To terminate the test: cultures were fixed in 10% formalin, the agar removed, the monolayers stained with 1% crystal violet and plaques counted. Each compound concentration was run in triplicate. Control wells (without virus) were observed for toxicity of compounds at the termination of each test and graded morphologically from 0 to 4. 0 is no toxicity observed while 4 is total lysing of the cell monolayer.

### 96 well plate assay

The 96 well plate assay was performed similarly to the plaque assay above with modifications. SCP cells were seeded at 1 x 10⁴ cells per well in 0.1 ml DME medium. When confluent, the wells were washed with serum free MEM and 25ul of virus added in M-199 supplemented with 2% lamb serum. After 1 hour, 75uL of medium containing test compound was added to each well containing virus. After 2-3 weeks incubation the cytopathic effect of the virus was determined by staining with a vital stain. Cell viability was measured by determining stain density using a 96 well plate reader.

Control wells without virus were completed to determine the toxicity of compounds.

### RESULTS

Table 1, below, sets forth the results of the assay for the compounds of Examples 3 and 5 compared to the N-butyl derivative of 1,5-dideoxy-1,5-imino-D-glucitol (N-Bu-DNJ) as a control standard.

**Table 1**

| PLAQUE REDUCTION ASSAY | | | | |
|---|---|---|---|---|
| Compound Example No. | Concentration mM | Toxicity | %Plaque Reduction | Antiviral Activity |
| N-Bu-DNJ | 1.0 | 2 | 100 | A |
| | 0.1 | 1 | 100 | A |
| | 0.01 | 0 | 13 | I |
| | 0.001 | 0 | -74 | I |
| | | | | |
| 3 | 1.0 | 0 | 90 | A |
| | 0.1 | 0 | 72 | A |
| | 0.01 | 0 | -64 | I |
| | 0.001 | 0 | -46 | I |
| | | | | |
| 5 | 1.0 | 0 | 83 | A |
| | 0.1 | 0 | 10 | I |
| | 0.01 | 0 | 10 | I |
| | 0.001 | 0 | 9 | I |

A = active compound; I = inactive

Toxicity graded on 0 to 4 scale; 0 = no toxicity and

4 = total cell lyses.

N-Bu-DNJ = n-butyl-deoxynojirimycin used as a control standard.

The EC₅₀ concentration (mM) for inhibition of visna virus for various compounds as prepared above is shown in the following Table 2:

**Table 2**

| Compound Example No. | EC₅₀ (mM) |
|---|---|
| 6 | 0.125 |
| 9 | 0.1 |
| 15 | 0.1 |
| 18 (comparative example) | 0.1 |
| 20 | 1.0 |
| 23 | 0.001 |
| 26 | 1.0 |
| 28 | 1.0 |

### Example 39

Various compounds as prepared above were tested for enzyme inhibitory activity against alpha- and beta-glucosidase enzymes as follows:

### ASSAYS FOR ALPHA-GLUCOSIDASE (YEAST) AND BETA-GLUCOSIDASE (ALMONDS)

Yeast alpha-glucosidase and almond beta-glucosidase activities were measured by a modification of the method of Evans, et al., Phytochemistry 22, 768-770 (1983). The modifications included 1) assay of activities at pH 7.4 in HEPES (N-2-hydroxyethylpiperazine-N'-2-ethanesulfonic acid) buffer, 2) measurement in 96 well microtiter plates and 3) inclusion of 10% DMSO in control and test samples.

The release of p-nitrophenol from the substrate p-nitrophenylglycoside was measured spectrophotometrically in the presence and absence of test compound. Each assay included a known inhibitor of the enzyme as a standard. IC₅₀ values were determined for compounds which inhibited the enzymes more than 50% at a 1 millimolar concentration.

### ALPHA-GLUCOSIDASE INHIBITION ASSAY, pH 7.4

To 100 µl 50 mM HEPES buffer, pH 7.4, in a microtiter plate, add 20 µl test compound in DMSO (DMSO alone in control), 40 µl (0.013 units) yeast alpha-glucosidase (Sigma) in HEPES buffer and pre-incubate at room temperature for 15 minutes. Add 40 µl 1.25 mM p-nitrophenyl-alpha-D-glucopyranoside (Sigma) in HEPES buffer, as substrate, and monitor absorbance change at 405 nm in a Biotek EIA Autoreader. Absorption change was measured at 15 to 25 minutes (reaction was linear for at least 30 minutes). Each sample was tested in triplicate. IC₅₀ values were determined from the linear portion of the log concentration vs % inhibition curve obtained from a minimum of 3 points. Deoxynojirimycin was used as standard inhibitor.

### BETA-GLUCOSIDASE INHIBITION ASSAY

### pH 7.4:

To 100 µl 50 mM HEPES buffer, pH 7.4, in a microtiter plate, add 20 µl test compound in DMSO (DMSO alone in control), 40 µl (0.136 units) beta-glucosidase (Sigma) in HEPES buffer and pre-incubate at room temperature for 15 minutes. Add 40 µl 1.25 mM p-nitrophenyl-beta-D-glucopyranoside in HEPES buffer, as substrate and monitor absorbance change at 405 nm in a Biotek EIA Autoreader. Absorption change was measured at 15 to 25 minutes (reaction is linear for at least 30 minutes). Each sample was tested in triplicate. IC₅₀ values were determined from the linear portion of the log concentration vs % inhibition curve obtained from a minimum of 3 points. Castanospermine was used as standard inhibitor.

### pH 4.8:

To 100 µl 50 mM sodium citrate buffer, pH 4.8, in a microtiter plate, add 15 µl test compound in DMSO (DMSO alone in control), 20 µl (.017 units) beta-glucosidase (Sigma) in citrate buffer and pre-incubate at room temperature for 15 minutes. Add 25 µl 2.50 mM p-nitrophenyl-beta-D-glucopyranside in citrate buffer, as substrate. Incubate at room temperature 20 minutes (reaction is linear for at least 30 minutes). Add 50 µl 0.4 M NaOH. Read absortion change at 405 nm in a Biotek EIA Autoreader. Each sample was tested in triplicate. IC₅₀ values were determined from the linear portion of the log concentration vs % inhibition curve obtained from a minimum of 3 points. Castanospermine was used as standard inhibitor.

The inhibitory activity against alpha- and beta-glucosidase enzymes by various compounds prepared above is shown in the following table 3:

**Table 3**

| Compound Example No. | Inhibitory Activity |
|---|---|
| 6 | α-glucosidase |
| | IC₅₀ = 35 µM |
| 8 | α-glucosidase |
| | IC₅₀ = 2.2 µM |
| | β-glucosidase |
| | IC₅₀ = >1000 µM |
| | at pH 4.8 |
| | and 7.4 |
| 16 | α-glucosidase |
| | IC₅₀ = 13 µM |
| | β-glucosidase |
| | IC₅₀ = >1000 µM |
| | at pH 4.8 |
| | and 7.4 |
| 18 (comparative example) | α-glycosidase |
| | IC₅₀ = 46 µM |
| | β-glucosidase |
| | IC₅₀ = 36 µM at pH 4.8 |
| | 23 µM at pH 7.4 |
| 27 | α-glucosidase |
| | 23% at 1 mM |

### Example 40

Further testing for A. inhibition of visna virus and B. enzyme inhibitory activity against alpha- and beta-glucosidase enzymes was carried out on various compounds as prepared, above, by the assay methods described in Examples 38 and 39, respectively. The results are shown in the following Tables 4 and 5:

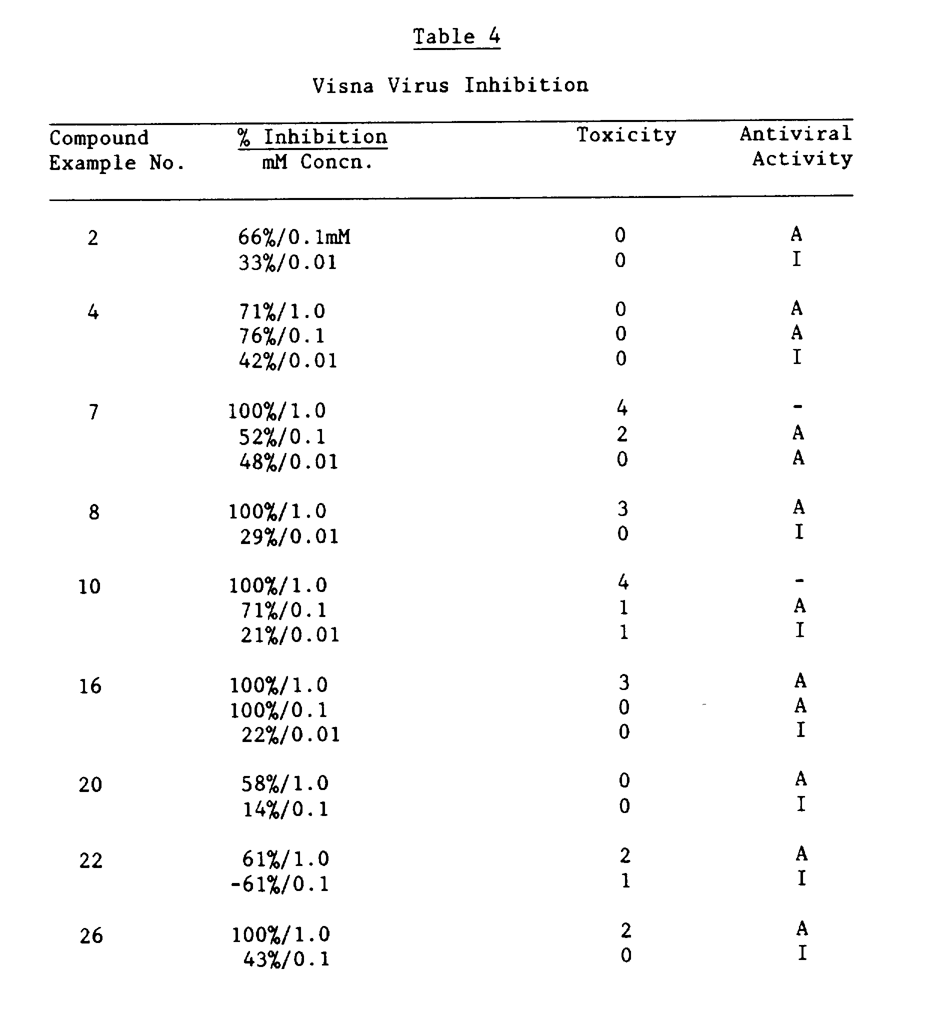

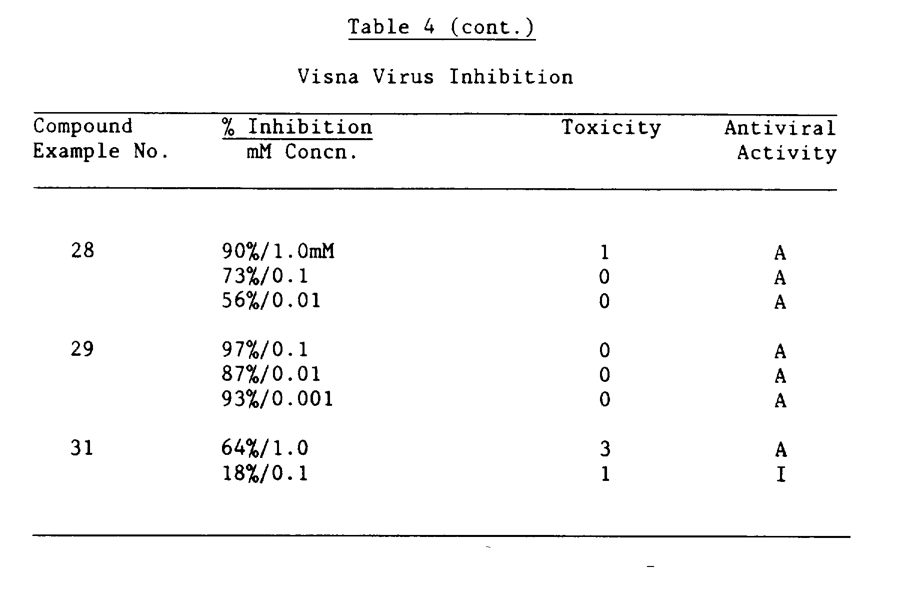

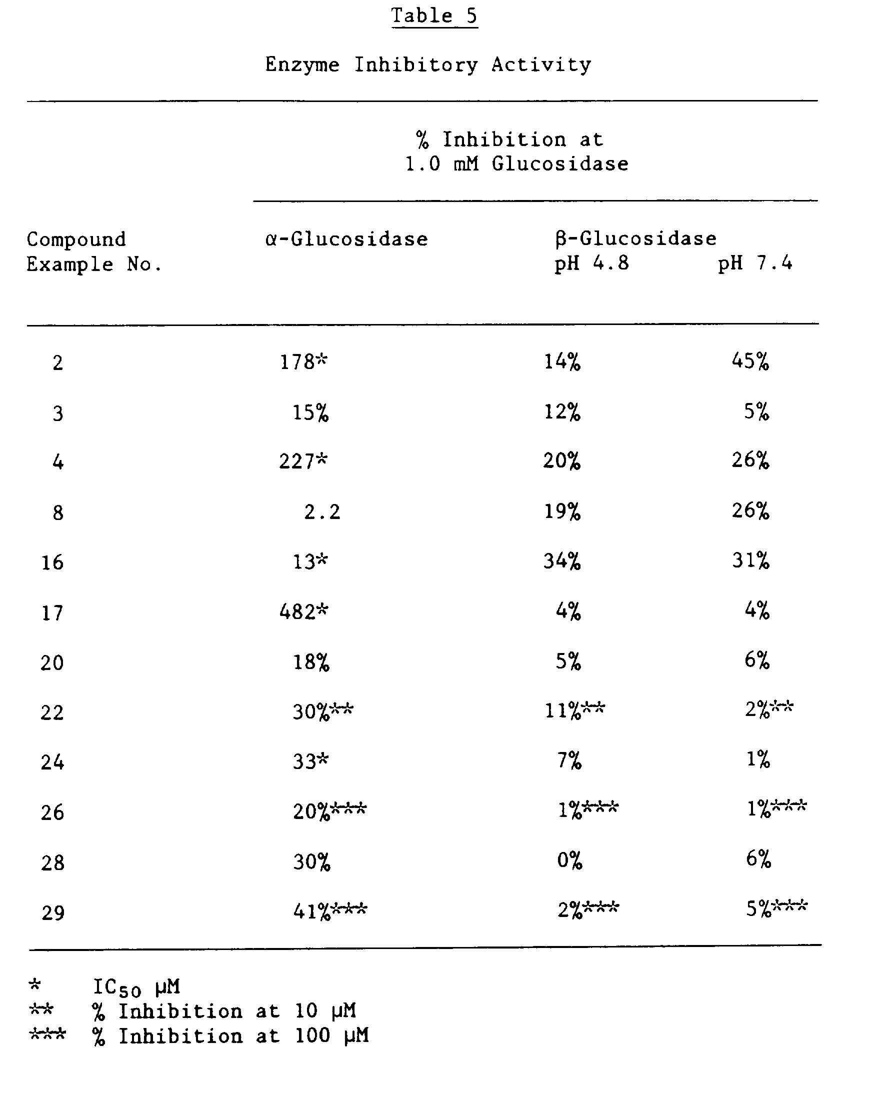

The antiviral agents described herein can be used for administration to a mammalian host infected with a virus, e.g. visna virus or the human immunodeficiency virus, by conventional means, preferably in formulations with pharmaceutically acceptable diluents and carriers. These agents can be used in the free amine form or in their salt form. Pharmaceutically acceptable salt derivatives are illustrated, for example, by the HCl salt. The amount of the active agent to be administered must be an effective amount, that is, an amount which is medically beneficial but does not present toxic effects which overweigh the advantages which accompany its use. It would be expected that the adult human dosage would normally range upward from about one milligram of the active compound. The preferable route of administration is orally in the form of capsules, tablets, syrups, elixirs and the like, although parenteral administration also can be used. Suitable formulations of the active compound in pharmaceutically acceptable diluents and carriers in therapeutic dosage form can be prepared by reference to general texts in the field such as, for example, Remington's Pharmaceutical Sciences, Ed. Arthur Osol, 16th ed., 1980, Mack Publishing Co., Easton, PA.

## Claims

1. A compound of the formula 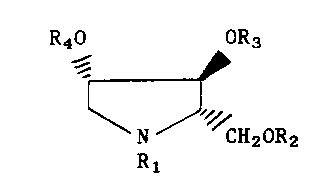
wherein R₁ is C₁-C₁₄ alkyl or C₁-C₁₄ hydroxyalkyl or C₃-C₁₂ alkanoyl or a substituted or unsubstituted aryl radical having six to ten carbon atoms; and
wherein R₂, R₃ and R₄ are the same or different and each is H or acyl having one to six carbon atoms; provided, however, that when R₂, R₃ and R₄ are each H, then R₁ is C₄-C₉ alkyl or C₂-C₅ hydroxyalkyl or C₃-C₁₂ alkanoyl.

2. A compound according to Claim 1 wherein R₁ is selected from the group consisting of alkylphenyl, alkyl-substituted alkylphenyl, halo-substituted alkylphenyl and benzyloxycarbonyl.

3. A compound according to Claim 1 wherein R₁ is selected from the group consisting of alkyl and hydroxyalkyl.

4. A compound according to Claim 1 wherein R₂, R₃ and R₄ are each the same acyl.

5. A compound according to Claim 2 wherein R₂, R₃ and R₄ are each the same acyl.

6. A compound according to Claim 3 wherein R₂*,* R₃ and R₄ are each the same acyl.

7. Compound according to claim 1 which is 1,4-(Phenylacetylimino)-1,4-dideoxy-L-arabinitol, triacetate.

8. Compound according to claim 1 which is 1,4-(Benzyloxycarbonylimino )-1,4-dideoxy-L-arabinitol, tripropionate.

9. Compound according to claim 1 which is 1,4-([2-Acetyloxyethyl]-imino)-1,4-dideoxy-L-arabinitol, triacetate.

10. Compound according to claim 1 which is 1,4-(Butylimino)-1,4-dideoxy-L-arabinitol, triacetate.

11. Compound according to claim 1 which is 1,4-(Butylimino)-1,4-dideoxy-L-arabinitol.

12. Compound according to claim 1 which is 1,4-(Hexylimino)-1,4-dideoxy-L-arabinitol.

13. Compound according to claim 1 which is 1,4-(Nonylimino)-1,4-dideoxy-L-arabinitol.

14. Componud according to claim 1 which is 1,4-([2-Hydroxyethyl]imino)-1,4-dideoxy-L-arabinitol.

15. Compound according to claim 1 which is 1,4-([5-Hydroxypentyl]imino)-1,4-dideoxy-L-arabinitol.

16. Compound according to claim 1 which is 1,4-(Propionylimino)-1,4-dideoxy-L-arabinitol.

17. Compound according to claim 1 which is 1,4-(2-Methylpropionylimino)-1,4-dideoxy-L-arabinitol.

18. Compound according to claim 1 which is 1,4-(Propionylimino)-1,4-dideoxy-L-arabinitol, triacetate.

19. Compound according to claim 1 which is 1,4-(2-Methylpropionylimino)-1,4-dideoxy-L-arabinitol, triacetate.

20. Use of a compound of the formula 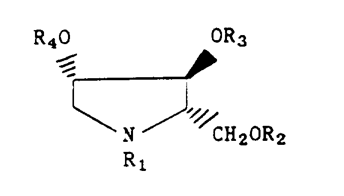
wherein R₁ is C₁-C₁₄ alkyl or C₁-C₁₄ hydroxyalkyl or C₃-C₁₂ alkanoyl or a substituted or unsubstituted aryl radical having six to ten carbon atoms; and
wherein R₂, R₃ and R₄ are the same or different and each is H or acyl having one to six carbon atoms; provided, however, that when R₂, R₃ and R₄ are each H, then R₁ is C₄-C₉ alkyl or C₂-C₅ hydroxyalkyl or C₃-C₁₂ alkanoyl,
or a pharmaceutically acceptable salt derivative of such a compound, for the manufacture of a medicament for administration to a mammalian host infected with a virus.

21. Use of a compound or derivative according to Claim 20 in which the virus is human immunodeficiency virus.

## Patentansprüche

1. Verbindung der Formel 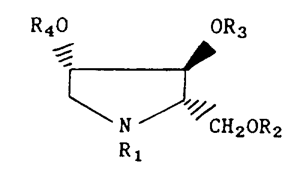
worin R₁ C₁-C₁₄-Alkyl oder C₁-C₁₄-Hydroxyalkyl oder C₃-C₁₂-Alkanoyl oder einen substituierten oder unsubstituierten Arylrest mit 6 bis 10 Kohlenstoffatomen bedeutet; und
worin R₂, R₃ und R₄ gleich oder verschieden sind und jeweils H oder Acyl mit 1 bis 6 Kohlenstoffatomen darstellen;
jedoch mit der Maßgabe, daß, wenn R₂, R₃ und R₄ jeweils H bedeuten, R₁ C₄-C₉-Alkyl oder C₂-C₅-Hydroxyalkyl oder C₃-C₁₂-Alkanoyl darstellt.

2. Verbindung nach Anspruch 1, worin R₁ ausgewählt ist aus der Gruppe bestehend aus Alkylphenyl, Alkyl-substituiertem Alkylphenyl, Halogen-substituiertem Alkylphenyl und Benzyloxycarbonyl.

3. Verbindung nach Anspruch 1, worin R₁ ausgewählt ist aus der Gruppe bestehend aus Alkyl und Hydroxyalkyl.

4. Verbindung nach Anspruch 1, worin R₂, R₃ und R₄ jeweils dasselbe Acyl bedeuten.

5. Verbindung nach Anspruch 2, worin R₂, R₃ und R₄ jeweils dasselbe Acyl bedeuten.

6. Verbindung nach Anspruch 3, worin R₂, R₃ und R₄ jeweils dasselbe Acyl bedeuten.

7. Verbindung nach Anspruch 1, welche 1,4-(Phenylacetylimino)-1,4-didesoxy-L-arabinittriacetat ist.

8. Verbindung nach Anspruch 1, welche 1,4-(Benzyloxycarbonylimino)-1,4-didesoxy-L-arabinittripropionat ist.

9. Verbindung nach Anspruch 1, welche 1,4-([2-Acetyloxyethyl]-imino)-1,4-didesoxy-L-arabinittriacetat ist.

10. Verbindung nach Anspruch 1, welche 1,4-(Butylimino)-1,4-didesoxy-L-arabinittriacetat ist.

11. Verbindung nach Anspruch 1, welche 1,4-(Butylimino)-1,4-didesoxy-L-arabinit ist.

12. Verbindung nach Anspruch 1, welche 1,4-(Hexylimino)-1,4-didesoxy-L-arabinit ist.

13. Verbindung nach Anspruch 1, welche 1,4-(Nonylimino)-1,4-didesoxy-L-arabinit ist.

14. Verbindung nach Anspruch 1, welche 1,4-([2-Hydroxyethyl]-imino)-1,4-didesoxy-L-arabinit ist.

15. Verbindung nach Anspruch 1, welche 1,4-([5-Hydroxypentyl]-imino)-1,4-didesoxy-L-arabinit ist.

16. Verbindung nach Anspruch 1, welche 1,4-(Propionylimino)-1,4-didesoxy-L-arabinit ist.

17. Verbindung nach Anspruch 1, welche 1,4-(2-Methylpropionylimino)-1,4-didesoxy-L-arabinit ist.

18. Verbindung nach Anspruch 1, welche 1,4-(Propionylimino)-1,4-didesoxy-L-arabinittriacetat ist.

19. Verbindung nach Anspruch 1, welche 1,4-(2-Methylpropionylimino)-1,4-didesoxy-L-arabinittriacetat ist.

20. Verwendung einer Verbindung der Formel 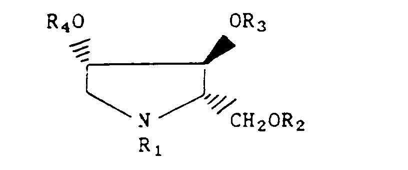
worin R₁ C₁-C₁₄-Alkyl oder C₁-C₁₄-Hydroxyalkyl oder C₃-C₁₂-Alkanoyl oder einen substituierten oder unsubstituierten Arylrest mit 6 bis 10 Kohlenstoffatomen bedeutet; und
worin R₂, R₃ und R₄ gleich oder verschieden sind und jeweils H oder Acyl mit 1 bis 6 Kohlenstoffatomen darstellen;
jedoch mit der Maßgabe, daß, wenn R₂, R₃ und R₄ jeweils H bedeuten, R₁ C₄-C₉-Alkyl oder C₂-C₅-Hydroxyalkyl oder C₃-C₁₂-Alkanoyl darstellt,
oder eines pharmazeutisch annehmbaren Salz-Derivats einer derartigen Verbindung, bei der Herstellung eines Medikaments zur Verabreichung an einem mit einem Virus infizierten Säuger-Wirt.

21. Verwendung einer Verbindung oder eines Derivats nach Anspruch 20, wobei das Virus das humane Immunschwächevirus ist.

## Revendications

1. Composé de formule 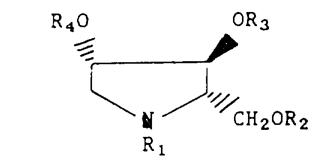
dans laquelle R₁ représente un groupe alkyle en C₁₋₁₄, hydroxyalkyle en C₁₋₁₄ ou alcanoyle en C₃₋₁₂, ou encore un groupe aryle comportant de 6 à 10 atomes de carbone, portant ou non des substituants, et
R₂, R₃ et R₄ sont identiques ou différents et représentent chacun un atome d'hydrogène ou un groupe acyle comportant de 1 à 6 atomes de carbone, sous réserve que, si R₂, R₃ et R₄ représentent chacun un atome d'hydrogène, R₁ représente un groupe alkyle en C₄₋₉ hydroxyalkyle en C₂₋₅ ou alcanoyle en C₃₋₁₂.

2. Composé conforme à la revendication 1, dans lequel R₁ est choisi dans l'ensemble que constituent les groupes alkyl-phényle, alkyl-phényle à substituant(s) alkyle, alkyl-phényle à substituant(s) halogéno, et benzyloxycarbonyle.

3. Composé conforme à la revendication 1, dans lequel R₁ est choisi dans l'ensemble que constituent les groupes alkyle et les groupes hydroxyalkyle.

4. Composé conforme à la revendication 1, dans lequel R₂, R₃ et R₄ représentent chacun le même groupe acyle.

5. Composé conforme à la revendication 2, dans lequel R₂, R₃ et R₄ représentent chacun le même groupe acyle.

6. Composé conforme à la revendication 3, dans lequel R₂, R₃ et R₄ représentent chacun le même groupe acyle.

7. Composé conforme à la revendication 1, qui est le triacétate de 1,4-(phénylacétyl-imino)- 1,4-didésoxy-L-arabitol.

8. Composé conforme à la revendication 1, qui est le tripropionate de 1,4-(benzyloxycarbonyl-imino)-1,4-didésoxy-L-arabitol.

9. Composé conforme à la revendication 1, qui est le triacétate de 1,4-([2-acétyloxyethyl]-imino)- 1 ,4-didésoxy-L-arabitol.

10. Composé conforme à la revendication 1, qui est le triacétate de 1,4-(butyl-imino)-1,4-didésoxy-L-arabitol.

11. Composé conforme à la revendication 1, qui est le 1,4-(butyl-imino)-1,4-didésoxy-L-arabitol.

12. Composé conforme à la revendication 1, qui est le 1,4-(hexylimino)-1,4-didésoxy-L-arabitol.

13. Composé conforme à la revendication 1, qui est le 1,4-(nonylimino)-1,4-didésoxy-L-arabitol.

14. Composé conforme à la revendication 1, qui est le 1,4-([2-hydroxyéthyl]-imino)-1,4-didésoxy-L-arabitol.

15. Composé conforme à la revendication 1, qui est le 1,4-([5-hydroxypentyl]-imino)-1,4-didésoxy-L-arabitol.

16. Composé conforme à la revendication 1, qui est le 1,4-(propionyl-imino)-1,4-didésoxy-L-arabitol.

17. Composé conforme à la revendication 1, qui est le 1,4-([2-méthylpropionyl]-imino)-1,4-didésoxy-L-arabitol.

18. Composé conforme à la revendication 1, qui est le triacétate de 1,4-(propionyl-imino)-1,4-didésoxy-L-arabitol.

19. Composé conforme à la revendication 1, qui est le triacétate de 1,4-(([2-méthylpropionyl]-imino)-1,4-didésoxy-L-arabitol.

20. Utilisation d'un composé de formule 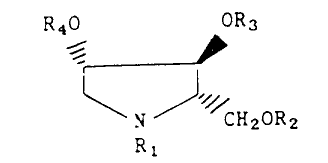
dans laquelle R₁ représente un groupe alkyle en C₁₋₁₄, hydroxyalkyle en C₁₋₁₄ ou alcanoyle en C₃₋₁₂, ou encore un groupe aryle comportant de 6 à 10 atomes de carbone, portant ou non des substituants, et
R₂, R₃ et R₄ sont identiques ou différents et représentent chacun un atome d'hydrogène ou un groupe acyle comportant de 1 à 6 atomes de carbone, sous réserve que, si R₂, R₃ et R₄ représentent chacun un atome d'hydrogène, R₁ représente un groupe alkyle en C₄₋₉ hydroxyalkyle en C₂₋₅ ou alcanoyle en C₃₋₁₂,
ou d'un sel acceptable en pharmacie qui est un dérivé d'un tel composé, pour la préparation d'un médicament destiné à être administré à un hôte mammifère infecté par un virus.

21. Utilisation d'un composé ou d'un dérivé, conforme à la revendication 20, dans laquelle le virus est un virus humain de déficit immunitaire.
